# EUROPEAN PATENT APPLICATION

(11) **EP 4 609 819 A1**
(43) Date of publication of application: **03.09.2025**
(21) Application number: 24160186.3
(22) Date of filing: 28.02.2024
(51) Int. Cl.: A61B 34/32, A61B 17/28, A61B 34/37, A61F 9/007, A61B 17/00, A61B 90/00

(54) **SURGICAL ROBOTIC SYSTEM AND CONTROL OF SURGICAL ROBOTIC SYSTEM**

(71) Applicant: Carl Zeiss Meditec AG, 07745 Jena (DE)
(72) Inventor: HILLENBRAND, Matthias, 73447 Oberkochen (DE); BRIEL, Marius, 76344 Eggenstein-Leopoldshafen (DE); HAIDE, Ludwig, 76344 Eggenstein-Leopoldshafen (DE); BEELEN, Maarten, 5612 AP Eindhoven (NL); DA COL, Tommaso, 5612 AP Eindhoven (NL)
(74) Representative: DeltaPatents B.V.

(57) **Abstract**

A surgical robotic system is provided for use in an intraocular procedure. The surgical robotic system comprises a surgical arm comprising a movable arm part for holding a surgical instrument, a human machine interface for receiving operator commands from a human operator, and a processor subsystem configured to control one or more actuators to control a pose of the surgical instrument based on the operator commands. The surgical robotic system may be configured to operate in a first operational mode in which the human operator is enabled to adjust the pose of the surgical instrument across multiple degrees of freedom, to switch from the first operational mode to a second operational mode in response to a trigger, and in the second operational mode, exert control over the pose of the surgical instrument across one or a subset of the degrees of freedom.

## Description

### TECHNICAL FIELD

The invention relates to a surgical robotic system for use in an intraocular procedure. The invention further relates to a method for controlling a surgical robotic system during an intraocular procedure, and to a computer program comprising instructions for causing a processor system to perform the method.

### BACKGROUND

Intraocular procedures increasingly involve the use of surgical robotic systems. Rather than operating entirely autonomously, such surgical robotic systems are expected for the foreseeable future to remain at least in part under the control of a human operator. For example, the human operator may directly or indirectly control the movement of a surgical instrument mounted to a surgical arm of the surgical robotic system. Nevertheless, it is expected that future surgical robotic systems may be more autonomous and require less or even no involvement of a human operator.

A surgical robotic system designed for intraocular procedures may be provided with a surgical arm which comprises a movable arm part, with the movable arm part comprising an end effector for holding a surgical instrument. Accordingly, the surgical instrument may be positioned by the surgical arm. An actuator subsystem may be provided for actuating the movable arm part to control a pose of the surgical instrument. Here, the term 'pose' may refer to a position and orientation of surgical instrument. Through such actuation, the surgical instrument may be moved in a lateral direction, for example relative to a retinal surface, and in a longitudinal direction along a longitudinal axis of the surgical instrument. Longitudinal movement may typically allow the surgical instrument to be moved towards and away from a surgical target within an interior of the eye. Accordingly, the surgical instrument may be used to modify (biological) tissue near the surgical target, to deliver an agent to the surgical target, etc. Examples of surgical instruments include, but are not limited to, forceps, mechanical cutters, coagulation cutters, scissors, injection needles, sealing devices, etc.

Surgical robotic systems of the above type are known per se. For example, WO 2016/030336 A1 describes a surgical robotic system for use in a surgical procedure, comprising a surgical arm comprising a movable arm part, the movable arm part comprising an instrument connector for mounting of a surgical instrument, the surgical instrument having a longitudinal axis, the movable arm part having at least one degree-of-freedom to enable longitudinal movement of the surgical instrument along the longitudinal axis of the surgical instrument towards a surgical target. The surgical robotic system is said to further comprise a human machine interface for receiving positioning commands from a human operator for controlling the longitudinal movement of the surgical instrument, an actuator configured for actuating the movable arm part to effect the longitudinal movement of the surgical instrument, and a processor configured for controlling the actuator in accordance with the positioning commands.

While the use of surgical robotic systems of the above type in intraocular procedures offers numerous benefits, including enhanced precision and dexterity, an issue is the potential for the human operator to unintentionally inflict damage within the eye to due to the human operator incorrectly controlling the surgical instrument's pose. The problem of incorrect control may be aggravated in cases where the human operator is granted extensive control over the pose of the surgical instrument. Namely, such extensive control may be challenging for the human operator to manage, particularly during moments of intense focus on executing specific surgical actions.

### SUMMARY

It may be advantageous to be able to address one or more of the aforementioned problems.

In accordance with a first aspect of the invention, a surgical robotic system is provided for use in a surgical procedure, such as an intraocular procedure, comprising:
- a surgical arm comprising a movable arm part, wherein the movable arm part comprises an end effector for holding a surgical instrument;
- an actuator subsystem configured to actuate the movable arm part;
- a human machine interface for receiving operator commands from a human operator;
- a processor subsystem configured to control the actuator subsystem to control a pose of the surgical instrument based on the operator commands;

wherein the surgical robotic system is configured to operate in a first operational mode in which the human operator is enabled to adjust the pose of the surgical instrument across multiple degrees of freedom by providing corresponding operator commands via the human machine interface and wherein the processor subsystem controls the actuator subsystem to effect said adjustment of the pose of the surgical instrument across the multiple degrees of freedom;
wherein the processor subsystem is further configured to switch from the first operational mode to a second operational mode in response to a trigger, and in the second operational mode, exert control over the pose of the surgical instrument across one or a subset of the degrees of freedom.

In accordance with a further aspect of the invention, a computer-implemented method is provided for controlling a surgical robotic system during a surgical procedure, such as an intraocular procedure, wherein the surgical robotic system comprises:
a surgical arm comprising a movable arm part, wherein the movable arm part comprises an end effector for holding a surgical instrument;
   - an actuator subsystem configured to actuate the movable arm part;
   - a human machine interface for receiving operator commands from a human operator;
   - a processor subsystem configured to control the actuator subsystem to control a pose of the surgical instrument based on the operator commands;
wherein the method comprises:
   - in a first operational mode of the surgical robotic system:
receiving operator commands from the human machine interface to adjust the pose of the surgical instrument across multiple degrees of freedom, and
controlling the actuator subsystem to effect said adjustment of the pose of the surgical instrument across the multiple degrees of freedom;
   - switching from the first operational mode to a second operational mode in response to a trigger;
   - in the second operational mode, exerting control over the pose of the surgical instrument across one or a subset of the degrees of freedom.

In accordance with a further aspect of the invention, a transitory or non-transitory computer-readable medium is provided comprising data representing a computer program, the computer program comprising instructions for causing a processor system to perform the above-identified computer-implemented method.

The above aspects of the invention provide a surgical robotic system which comprises a surgical arm. The surgical arm may comprise a movable arm part, and the movable arm part may comprise an end effector for holding a surgical instrument. For example, the end effector may comprise an instrument connector for mounting the surgical instrument, e.g., in a removable manner. The surgical instrument may be configured to enter a patient's interior, e.g., through an incision, a trocar, or a natural orifice. The surgical instrument may typically have a longitudinal axis which may pass through a tip of the surgical instrument. The movable arm part may have multiple degrees of freedom (DOF). Using these multiple DOFs, the tip of the surgical instrument may typically be moved longitudinally, for example to advance towards and/or retract away from a surgical target, and laterally along a plane perpendicular to the longitudinal axis. In general, the movable arm part may have sufficient DOFs to enable the surgical instrument to be adjustable in pose, e.g., in position and orientation. It is noted that surgical arms having the functionality described in this paragraph are known per se from the field of medical robotics, and also known as instrument manipulators, robotic arms, surgical robot slave devices, etc.

An actuator subsystem may be provided for actuating the movable arm part to adjust the pose of the movable arm part, and thereby to adjust the pose of the surgical instrument. Another term for actuator subsystem is driving mechanism.

A human machine interface may be provided for enabling a human operator to provide operator commands to the surgical robotic system, and a processor subsystem may be provided which is configured to control the actuator subsystem to control a pose of the surgical instrument based on the operator commands. Such type of control may also be referred to as master-slave control. Using such master-slave control, the human operator may exert control over the pose of the surgical instrument.

In accordance with the above measures, the degree of control of the human operator may be limited in some instances. Namely, the processor subsystem may be configured to operate in a first operational mode and in a second operational mode and may switch from the first operational mode to the second operational mode in response to a trigger, for example an internal or external trigger. In the first operational mode, the human operator may be enabled to adjust the pose of the surgical instrument across multiple degrees of freedom, while in the second operational mode, the processor subsystem may exert control over the pose of the surgical instrument across one or a subset of the degrees of freedom, for example by limiting the adjustment of the pose by the human operator across the one or the subset of the degrees of freedom. Such limitation of the adjustment of the pose may for example comprise reducing a range and/or direction of movement or even disallowing movement in a certain direction or along a certain axis. Alternatively to the movement, or additionally, such limitations may be applied to control parameters pertaining to acceleration, relative or absolute position, relative or absolute orientation, etc. Accordingly, in the first operational mode, the human operator may adjust the pose across *N* degrees of freedom, while in the second operational mode, the control by the human operator may be limited or entirely disallowed in a subset *M* of these *N* degrees of freedom, e.g., in *M* ⊆ *N* with *M* ≥ 1.

It is noted that different DOFs in the subset *M* may be subject to different limitations. For example, for the subset *M* comprising {*DOF*₁, *DOF*₂, ..., *DOF*_{M}}, human operator control may be disallowed in *DOF*₁ while limitations may be applied in *DOF*₂. As such, when considering the control space available to the human operator, for example represented by a range or set of allowed control parameters for each of the DOFs, the control space in the second operational mode may be a subset, or in general be smaller in size, than the control space in the first operational mode. When the processor subsystem operates in the second operational mode, the control of the human operator over the pose of the surgical instrument may thus be more restricted than when the processor subsystem operates in the first operational mode.

In this respect, it noted that individual DOFs in *N* and *M* may correspond to individual DOFs of actuators, but may also represent DOFs in a different coordinate system, e.g., a coordinate system used for the control of the surgical instrument.

The above measures have the effect that during a surgical procedure, such as an intraocular procedure, the processor subsystem, and thereby the surgical robotic system as a whole, may limit the master-slave control by the human operator in some instances. This may reduce the risk of unintentionally inflicting damage within the patient's interior, for example within the eye. Namely, by reducing the number of DOFs which are available for the control by the human operator (e.g., from *N* to *N\M*) and/or limiting the control of the human operator in one or more of the available DOFs *N*, the extent of control (e.g., in terms of dimensionality and/or size of the control space) may be reduced, which in turn reduces the chance of the human operator unintentionally adjusting the pose in a manner in which damage is inflicted within the patient's interior. There may be several factors contributing to such reduced risk. One factor may be that restricting the extent of control by a human operator inherently lowers the likelihood of such control causing harm inside the patient's interior. Another factor may be that if the extent of control by a human operator is limited, the human operator may only have to focus his/her attention to the more limited set of DOFs *N\M*, which in turn may reduce the complexity the control of the surgical instrument and thereby allow the human operator to control the surgical instrument in a more accurate and safe manner. A thereto related advantage may be that the pose of the surgical instrument may be controlled by the human operator in a less-complex, simpler manner. Yet another factor is that if the processor subsystem exerts control over the DOFs *M*, the processor subsystem may exert this control using (semi-)automatic techniques, for example a control based on sensor data, which may be safer than manual control.

The following optional aspects are described with reference to the surgical robotic system but may equally apply to the computer-implemented method for controlling the surgical robotic system and to the corresponding computer program.

Optionally, the processor subsystem is configured to exert the control by limiting the adjustment of the pose by the human operator across the one or the subset of the degrees of freedom. For example, the processor subsystem may be configured to limit the adjustment of the pose by the human operator across the one or the subset of the degrees of freedom by disallowing said adjustment by the human operator or by limiting the adjustment to remain within a range. For example, the range may be defined as a positional range, e.g., in absolute or relative terms, or as an orientational range, e.g., in absolute or relative terms, or as movement or acceleration range, etc.

Optionally, the processor subsystem is configured to limit the adjustment of the pose by the human operator to remain within the range by dampening the adjustment across the one or the subset of the degrees of freedom, for example by dampening excursions away from a reference point within the range or by dampening excursions beyond the range. For example, the reference point may be a midpoint of the range. By dampening the adjustment, it may be avoided that the human operator experiences an abrupt limitation in the control of the surgical instrument, which may otherwise be experienced as sudden and disruptive.

Optionally, the processor subsystem is configured to generate and output a sensory perceptible feedback signal which is indicative of the pose of the surgical instrument in relation to the range, such as an audible signal, a haptic signal, and/or a visual signal. The sensory perceptible feedback signal may guide the human operator to remain within the allowed range, thereby avoiding or reducing the chance of the human operator experiencing abrupt limitations in the control of the surgical instrument or otherwise having to be automatically guided back into the allowed range.

Optionally, the processor subsystem is configured to determine an extent to which to limit the adjustment of the pose by the human operator based on a relative position and/or relative orientation between the surgical instrument and an anatomical structure, such as an intraocular structure, for example the retina. The limitation that is applied to one or more of the DOFs, being for example a reduction in range or a general disallowance to adjust the pose along a respective DOF, may be dependent on a relative position and/or orientation with respect to an anatomical structure. Namely, certain positions and/or orientations of the surgical instrument relative to the anatomical structure may increase the risk of a human operator unintentionally inflicting damage onto the anatomical structure. Therefore, in the second operational mode, the limitation which is to be applied may be dependent on the position and/or orientation of the surgical instrument relative to the anatomical structure, for example to apply one or more control limitations, or greater control limitations, in a vicinity of the anatomical structure. Moreover, at certain relative positions and/or orientations of the surgical instrument to the anatomical structure, the human operator may not need all degrees of freedom available in the first operational mode, and accordingly, a limitation may be applied to enable the human operator to control the surgical instrument in a simpler manner, e.g., with less control complexity.

Optionally, the one or the subset of the degrees of freedom comprise one or more positional degrees of freedom, and the processor subsystem is configured to exert control over a position of the surgical instrument across the one or more positional degrees of freedom, for example to control the surgical instrument to remain within a positional range, or to follow a trajectory, or to remain within a positional range of the trajectory. The position of the surgical instrument to the anatomical structure may be of relevance in the surgical procedure. For example, in a retinal membrane peeling procedure, after grasping the retinal membrane with the surgical instrument, the surgical instrument may be moved predominately laterally to pull the retinal membrane loose from the retina but without applying excessive force or excessive traction on the retina and without colliding with the retina. In such and various other scenarios, the processor subsystem may exert a degree of control over the position of the surgical instrument and thereby remove that degree of control from the human operator, for example to improve safety or to support a surgical action performed with the surgical instrument. For example, in the retinal membrane procedure, the processor subsystem may switch or be switched to the second operational mode after grasping the retinal membrane, after which the position of the surgical instrument may be constrained to a range which avoids the aforementioned excessive force/traction and collisions, for example by limiting the longitudinal position of the surgical instrument. In general, the processor subsystem may be configured to control the surgical instrument to remain within the positional range, or to follow the trajectory, or to remain within the positional range of the trajectory, during various surgical procedures, including but not limited to intraocular procedures such as vitrectomy, retinal peeling, phacoemulsification, capsulotomy, glaucoma stand placement, subretinal injection, and retinal vein cannulation, ontological procedures such as the delivery of an cochlear implant, laryngeal procedures such as laser microsurgery in the vocal cord region, etc.

Optionally, the one or the subset of the degrees of freedom comprise one or more orientational degrees of freedom, and the processor subsystem is configured to exert control over an orientation of the surgical instrument across the one or more orientational degrees of freedom, for example to control the orientation of the surgical instrument relative to an anatomical structure, such as an intraocular structure. The orientation of the surgical instrument may be of relevance in the surgical procedure. For example, when using a surgical instrument in form of a micro vacuum pick to grasp a retinal surface structure, it may be desirable to, after grasping the retinal surface structure, keep the aspiration port of the micro vacuum pick aligned with the retinal surface structure. As such, after grasping the retinal surface structure, the processor subsystem may keep the micro vacuum pick aligned with the retinal surface structure, or may assist the human operator in keeping such alignment, e.g., by limiting or disallowing the ability of the human operator to rotate the aspiration port away from the retinal surface structure. The processor subsystem may thus exert a degree of control over the orientation of the surgical instrument, for example to improve safety or to support a surgical action.

Optionally, the processor subsystem is configured to access pre-operative and/or intraoperative sensor data, and based on the sensor data, control a distance and/or orientation of the surgical instrument relative to an anatomical structure, such as an intraocular structure, for example across one or more positional degrees of freedom and/or one or more orientational degrees of freedom. The processor subsystem may be configured to, in the second operational mode, exert a degree of control over the distance and/or orientation of the surgical instrument relative to the anatomical structure and thereby remove that degree of control from the human operator. To be able to determine the relative distance and/or orientation to the anatomical structure, the processor subsystem may access and analyse sensor data, for example real-time intraoperative sensor data or preoperative sensor data. For example, optical coherence tomography (OCT) sensor data may be used from an intra- or extraocular OCT sensor.

Optionally, the processor subsystem is configured to:
- access a computational model of an anatomical structure, such as an intraocular structure, which is pertinent in the surgical procedure, wherein the computational model is a geometric and/or a biomechanical model of the anatomical structure; and
- exert the control across the one or the subset of the degrees of freedom based on the computational model.

An anatomical structure may be of relevance in the surgical procedure. For example, the anatomical structure may represent a surgical target or may be a neighbouring structure to the surgical target. It may be desirable to, in the second operational mode, exert a degree of control over the surgical instrument in relation to the anatomical structure, for example when the anatomical structure is nearby, or after the anatomical structure is grasped by the surgical instrument, etc. The degree and/or type of control may depend on one or more geometric and/or biomechanical properties of the anatomical structure. To enable the processor subsystem to use the geometric and/or biomechanical properties of the anatomical structure in the control of the surgical instrument, the processor subsystem may access and use a computational model of the anatomical structure. The computational model may for example be a geometric model of the retina which defines a curvature of the retina and which may be used by the processor subsystem to limit the human operator's control over the pose of the surgical instrument to avoid collision with the retina. The degree of control exerted over the surgical instrument by the processor subsystem may thus be governed by geometric and/or biomechanical properties derived from the computational model.

Optionally, the processor subsystem is configured to access pre-operative and/or intraoperative sensor data and to calibrate the computational model using the sensor data. The computational model may thus be initialized and/or updated based on the sensor data, thereby fitting the model to the anatomical structure at hand.

Optionally, the processor subsystem is configured to use the computational model during the surgical procedure to limit a magnitude of a force, or a directional component of the force, or a direction of the force, which is exerted by the surgical instrument onto or via the anatomical structure. Such limitations may for example be relevant in retinal membrane peeling procedures to reduce the risk of tearing.

Optionally, the processor subsystem is configured to switch from the first operational mode to the second operational mode in response to at least one of:
- a surgical action being performed with the surgical instrument, such as a grasping action, a suction action, or a cutting action;
- a gesture being performed with the surgical instrument;
- switching logic indicating when to switch to the second operational mode, wherein the switching logic is configured to use a computational model of an anatomical structure which is pertinent in the surgical procedure and evaluate the computational model to determine when to switch to the second operational mode.

The switch to the second operational mode may be triggered internally, e.g., by internal logic of the processor subsystem, or externally, e.g., in response to a trigger provided by the user. For example, it may be desirable to exert a degree of control over the surgical instrument and thereby remove that degree of control from the human operator during specific surgical steps, for example after a surgical action has been performed, or when a computational model, such as a geometric model or a biomechanical model, indicates that such a switch is desirable. Also the type of surgery may have an influence on the switching as the surgeon may want to have a higher level of assistance for simple cases while preferring to do it mostly manually for more complex case. Another example is that the human operator may trigger the switch, for example by performing a gesture with the surgical instrument, or by activating certain functionality of the surgical instrument. The switch may be also triggered via a user interface, or a selectable user profile for specific case types or based on a detected surgery phase.

Optionally, the processor subsystem is configured to switch from the first operational mode to the second operational mode in response to an operator command which comprises a request to switch or which is otherwise indicative of a need to switch. The human operator may thus directly or indirectly trigger the processor subsystem to switch to the second operational mode. An example of a direct trigger is the activation of a user interface element, e.g., a button, or another type of direct command. An example of an indirect trigger is an action which has another primary goal but which may also trigger the switching to the second operational mode.

Optionally, the surgical robotic system may be configured for microsurgery. The surgical instrument may thus be a microsurgical instrument.

Optionally, the surgical robotic system may be configured for minimally invasive surgery. The surgical instrument may thus be a surgical instrument for use in such minimally invasive surgery.

Optionally, the surgical robotic system may be configured for intraocular surgery. The surgical instrument may thus be an intraocular surgical instrument. Any references to anatomical features may thus be references to intraocular features.

It will be appreciated by those skilled in the art that two or more of the above-mentioned embodiments, implementations, and/or aspects of the invention may be combined in any way deemed useful.

Modifications and variations of the computer-implemented method and/or the computer-readable medium, which correspond to the described modifications, variations, and optional aspects of the surgical robotic system, may be carried out by a person skilled in the art on the basis of the present description.

### BRIEF DESCRIPTION OF THE DRAWINGS

These and other aspects of the invention are apparent from and will be elucidated with reference to the embodiments described hereinafter. In the drawings,
Fig. 1 shows a schematic representation of a surgical robotic system;
Fig. 2 shows a surgical instrument passing through a trocar during minimally invasive surgery, the surgical instrument having four degrees of freedom (DOFs);
Fig. 3 shows a joint diagram illustrating the kinematics of a movable arm part of a surgical arm for use in minimally invasive surgery;
Fig. 4 shows a joint diagram illustrating the kinematics of a motion controller;
Fig. 5 shows a surgical instrument in an eye according to an embodiment of the invention;
Fig. 6A shows a surgical instrument with a grasping device at an initial distance relative to a retinal surface structure;
Fig. 6B shows the surgical instrument with its grasping device having been advanced to reach a target distance with respect to the retinal surface structure;
Fig. 6C shows the grasping device having been operated to grasp the retinal surface structure;
Fig. 6D shows a retreating movement of the grasping device;
Fig. 6E shows an operator-controlled peeling movement being limited to remain within a longitudinal positional range;
Fig. 6F shows an operator-controlled peeling movement being limited to remain within a lateral positional range;
Fig. 7 schematically shows a method for controlling a surgical robotic system during a surgical procedure; and
Fig. 8 shows a non-transitory computer-readable medium;

It should be noted that items which have the same reference numbers in different figures, have the same structural features and the same functions, or are the same signals. Where the function and/or structure of such an item has been explained, there is no necessity for repeated explanation thereof in the detailed description.

### List of reference numerals

The following list of references and abbreviations is provided for facilitating the interpretation of the drawings and shall not be construed as limiting the claims.
- 20: human machine interface
- 22: operator commands
- 30: sensor
- 32: sensor data
- 40: processor subsystem
- 42: actuation commands
- 60: actuator subsystem
- 62: actuation of surgical arm
- 80: surgical arm
- 82: movable arm part
- 100: surgical robotic system
- 104: *e̅ₓ*, axis of coordinate system fixed to the instrument tip, orthogonal to the instrument longitudinal axis
- 105: *e̅_{y},* axis of coordinate system fixed to the instrument tip, orthogonal to the instrument longitudinal axis
- 106: *e̅_{z}*, axis of a cartesian coordinate system, aligned with the instrument longitudinal axis
- 107: *ϕ*, rotation of surgical instrument, laterally displacing its tip
- 108: *ψ*, rotation of surgical instrument, laterally displacing its tip
- 109: *z*, longitudinal (along its longitudinal axis) translation of surgical instrument, or penetration direction, or advancing direction
- 110: θ, rotation of surgical instrument around its longitudinal axis
- 111: *φ*, rotational DOF of a movable arm part
- 112: *ψ*, rotational DOF of a movable arm part
- 113: *z*, translational DOF of a movable arm part
- 114: *θ*, rotational DOF of a movable arm part
- 115: *φₘ,* rotational DOF of motion controller
- 116: *ψₘ*, rotational DOF of motion controller
- 117: *zₘ*, translational DOF of motion controller
- 118: *θₘ*, rotational DOF of motion controller
- 119: surgical instrument
- 121: sensor or sensor part
- 122: grasping device
- 123: surgical target
- 124: trocar
- 125: remote centre of motion (RCM)
- 126: button on motion controller gripper
- 127: optical beam
- 200: eye
- 210: retina
- 220: first layer of tissue
- 230: second layer of tissue
- 240: retinal surface structure
- 300: positioning grasping device relative to retinal surface structure
- 310: actuation of grasping mechanism
- 320: retreating movement
- 330: peeling movement
- 350: longitudinal positional range
- 360: lateral positional range
- 400: method of controlling surgical robotic system
- 410: processor subsystem operating in first operational mode
- 412: triggered transition to second operational mode
- 420: processor subsystem operating in second operational mode
- 422: triggered transition to first operational mode
- 500: non-transitory computer readable medium
- 510: data representing computer program

### DESCRIPTION OF EMBODIMENTS

The following embodiments relate to a surgical robotic system for use in a surgical procedure. The surgical robotic system may comprise a movable arm part. The movable arm part may comprise an end effector for holding a surgical instrument. The surgical robotic system may further comprise an actuator subsystem configured to actuate the movable arm part. During the surgical procedure, the surgical robotic system may operate in a master-slave mode in which the actuator subsystem may be controlled to adjust a pose of the surgical instrument based on the operator commands received from a human operator. More specifically, the surgical robotic system may operate in accordance with one of at least two operational modes. In a first operational mode, the human operator may be enabled to adjust the pose of the surgical instrument across multiple degrees of freedom by providing corresponding operator commands via a human machine interface. In a second operational mode, the surgical robotic system may exert control over the pose of the surgical instrument across one or a subset of these degrees of freedom. The surgical robotic system may switch from the first operational mode to the second operational mode in response to a trigger.

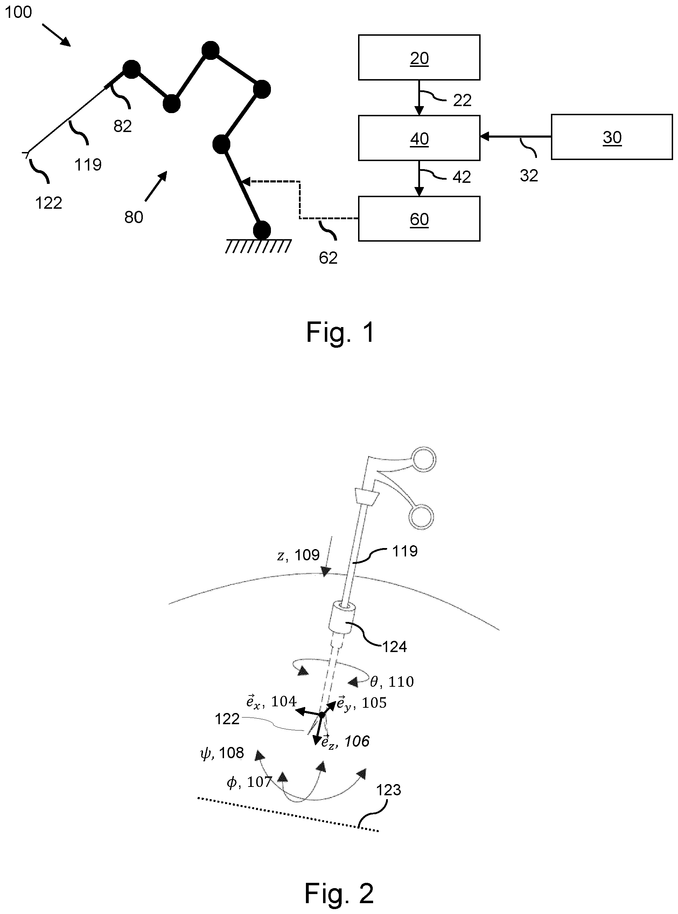

The following describes these operational modes within the context of a specific surgical robotic system, namely a surgical robotic system which is configured for intraocular surgery and described with reference to, inter alia, Figs. 1-6E. Nonetheless, the embodiments described in this specification are not confined solely to this type of surgical robotic system, nor to the field of intraocular surgery. Rather, the embodiments can be adapted and applied to other types of surgical robotic systems for use in intraocular procedures, as well as to other types of surgical robotic systems for use in other types of surgical procedures, such as microsurgical procedures, minimally invasive procedures, endoluminal procedures, etc. Any references to intraocular surgery and intraocular structures are therefore to be understood as applying also to other types of surgical procedures and corresponding types of anatomical structures.

Fig. 1 schematically shows a surgical robotic system 100 for use in an intraocular surgical procedure. The surgical robotic system 100 may comprise a surgical arm 80. The surgical arm 80 may comprise a movable arm part 82 which may comprise an end effector (not explicitly shown in Fig. 1) for holding a surgical instrument 119. To hold the surgical instrument 119, the end effector may comprise an instrument connector to which the surgical instrument 119 may be mounted. In the example of Fig. 1 and following, the surgical instrument 119 is shown to comprise a grasping device 122, such as a forceps or a micro vacuum pick. It will be appreciated, however, that the presently disclosed measures are not limited to surgical instruments which comprise or represent grasping devices, but rather apply to various types of surgical instruments, including those which comprise one or more other intraoperative devices. The grasping device 122 may be actuatable to perform a grasping action. The actuation may for example be mechanical, electrical, pneumatically, or otherwise.

The movable arm part 82 may have at least three degrees-of-freedom (DOF), and preferably at least six DOFs, to enable adjustment of the pose of the surgical instrument. For example, the movable arm part 82 may have DOFs to enable longitudinal movement of the surgical instrument towards a surgical target within the eye and lateral movement of the surgical instrument in a plane normal to said longitudinal movement. Here, longitudinal movement may refer to a movement of the surgical instrument 119 along its longitudinal axis (not separately shown in Fig. 1). In some embodiment, the surgical robotic system 100 may be configured to enable lateral movement. The lateral movement may be in a curved plane. The curvature of the curved plane may for example follow a curvature of the interior of the eye. Such type of lateral movement along a curved plane may be obtained by the surgical robotic system adjusting the longitudinal position of the surgical instrument 119 during lateral movement. The shape of the curved plane may for example be determined based on a model of the eye or an intraocular structure, e.g., the retina. The model may for example be a geometric model defined by parameters. The geometric model may be an example of a computational model as described elsewhere in this specification.

The surgical robotic system 100 may further comprise a human machine interface 20 for receiving operator commands 22 from a human operator. The operator commands 22 may for example comprise positioning commands from a human operator, e.g., a surgeon, to enable the human operator to determine a trajectory of the surgical instrument. In some embodiments, the positioning commands may be positioning commands for directly controlling the movement of the surgical instrument. Other types of operator commands may for example include confirmation inputs indicating instructions to continue with a procedure or procedural step, and/or input information, such as indicating an operating parameter and/or an indication of a surgical target position or the like. Examples of human machine interfaces for receiving operator commands include, but are not limited to, a keyboard, mouse, touch-sensitive surface, joystick, foot pedal, microphone, gesture recognition system, etc. The human machine interface may employ any suitable input modality, such as touch, push-actions, voice commands, eye movements, gesture recognition, etc.

The surgical robotic system 100 may further comprise an actuator subsystem 60 configured and arranged for actuating the movable arm part to effect the longitudinal movement and lateral movement of the surgical instrument. The actuator subsystem 60 may comprise any suitable actuator(s), e.g., from the field of surgical robots, or from the more general field of actuators. In particular, the actuator subsystem 60 may comprise a plurality of actuators which together provide the actuation of the movable arm part 60. Accordingly, a reference to a specific configuration of the actuator subsystem 60 may be understood as referring to a (joint) configuration of the plurality of actuators. Fig. 1 shows the actuation of surgical arm 80 schematically as a dashed line 62. It is noted that, although shown separately of the surgical arm 80, the actuator subsystem 60 may be integrated into, or mounted to, the surgical arm 80.

The surgical robotic system 100 may further comprise a sensor 30 which may be configured to generate sensor data which is indicative of a distance between the grasping device and an intraocular structure, such as the retina. For example, the sensor 30 may be or comprise an optical coherence tomography (OCT) probe. The OCT probe may for example be an optical fibre which is attached to or integrated in the surgical instrument 119, with the optical fibre being connected to a remotely located OCT sensor. In other examples, the OCT probe may comprise an OCT sensor which is directly attached to or directly integrated in the surgical instrument 119. The sensor data 32 provided by the OCT probe may comprise A-scans, which may comprise line measurements and defined as an intensity as a function of the distance, B-scans forming a 2D image, C-scans, e.g., from multiple B-scans, or the like. It is noted that even though the sensor 30 is shown in Fig. 1 to be separate from the surgical instrument 119, the sensor or part of the sensor may be attached to or integrated in the surgical instrument 119. In other examples, the sensor 30 may, in addition or alternatively to the OCT probe, comprise a stereo camera arranged for image capture through a microscope, an optical interferometric sensor integrated in or attached to the surgical instrument 119, a time-of-flight sensor integrated in or attached to the surgical instrument and an ultrasonic sensor integrated in or attached to the surgical instrument, a force-based sensor integrated in or attached to the surgical instrument, or an impedance or admittance sensor integrated in or attached to the surgical instrument. Although not shown in Fig. 1, in yet other examples, the surgical robotic system may comprise one or more extraocular sensors, e.g., an extraocular OCT sensor, or an additional intraocular sensor, e.g., an intraoperative impedance or admittance sensor. In some embodiments, the surgical robotic system 100 may comprise a microscope which may be communicatively coupled to the surgical robotic system 100.

The surgical robotic system 100 may further comprise a processor subsystem 40 configured for controlling the actuator subsystem 60 to control a pose of the surgical instrument 119 during the intraocular procedure. For the purpose of controlling the actuator subsystem 60, the processor subsystem 40 may provide actuation commands 42 to the actuator subsystem. Thereby, the processor subsystem 40 may adjust a position and orientation of the grasping device 122 during the intraocular procedure. As also elucidated elsewhere, the processor subsystem 40 may function in a master-slave mode, e.g., an operator control mode, in which the processor subsystem 40 directly carries out positioning commands received from a human operator. The processor subsystem 40 may also be configured to operate in an autonomous control mode in which the processor subsystem 40 autonomously controls the position of the surgical instrument 119, e.g., according to a pre-planned trajectory and sensor data, or in a semi-autonomous control mode which combines aspects of the operator control mode and the autonomous control mode. Such a semi-autonomous control mode may for example comprise parts of the intraocular procedure being carried-out under direct control of the human operator, e.g., 'manually', while other parts of the intraocular procedure may be carried out without such direct control.

When operating in the operator control mode, or in an operator-controlled phase when using the semi-autonomous control mode, the processor subsystem 40 may operate in one of at least two operational modes. In the first operational mode, the human operator may be enabled to adjust the pose of the surgical instrument across multiple degrees of freedom by providing corresponding operator commands via the human machine interface 20 and by the processor subsystem 40 controlling the actuator subsystem 60 to effect said adjustment of the pose of the surgical instrument 119 across the multiple degrees of freedom. In the second operational mode, the processor subsystem 40 may exert control over the pose of the surgical instrument 119 across one or a subset of these degrees of freedom. For example, the processor subsystem 40 may exert the control by limiting the adjustment of the pose by the human operator across the one or the subset of the degrees of freedom, which may comprise disallowing said adjustment by the human operator or limiting the adjustment to remain within a range. The processor subsystem 40 may switch from the first operational mode to the second operational mode in response to a trigger.

**Fig. 2** shows a surgical instrument 119 passing through a trocar 124 during minimally invasive surgery. For example, in case of vitreoretinal surgery, the trocar 124 may be placed in the sclera. Rotating around and translating through the trocar may be possible in four DOFs, e.g., the rotations *ϕ* 107, *ψ* 108, *θ* 110 and the translation *z* 109 to approach or penetrate a surgical target 123. Further shown are a tip 122 of the surgical instrument 119 and three axes 104-106 of a coordinate system fixed to the instrument tip 122, with *e̅_{z}* 106 aligned with the longitudinal axis of the surgical instrument 119. Rotations *ϕ* 107 and *ψ* 108 may result in a lateral displacement of the instrument tip 122, respectively in the direction *e̅_{y}* 105 and in the direction *e̅ₓ* 104. The translation z 109 may result in a longitudinal movement of the surgical instrument tip 122. The surgical robotic system 100 may be used in an intraocular surgical procedure, such as a minimally invasive surgical procedure as described above.

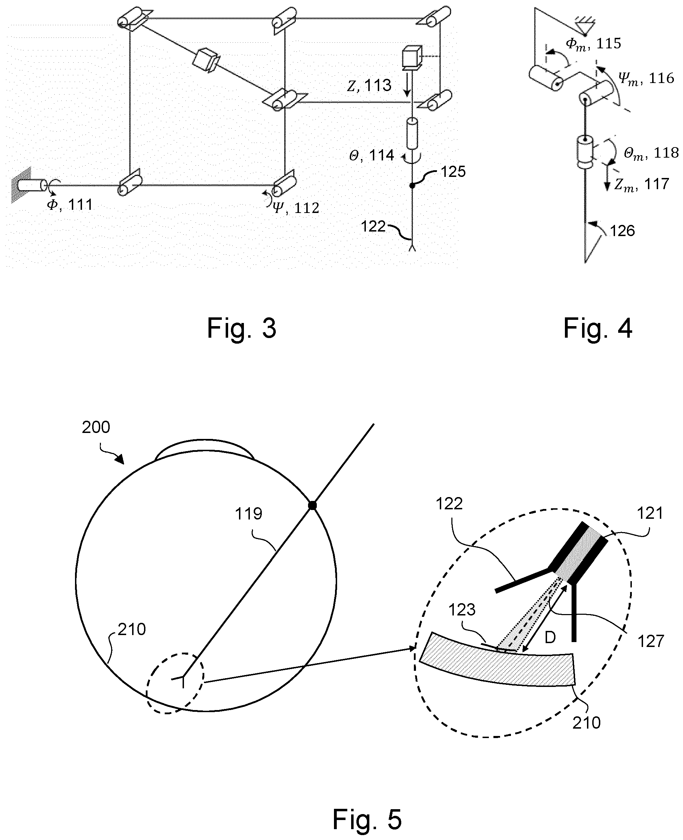

**Fig. 3** shows a joint diagram illustrating the kinematics of a movable arm part of a surgical arm for use in an intraocular surgical procedure, and in particular a minimally invasive surgery. In the example of Fig. 3, the surgical robotic system comprises a surgical arm, with the surgical arm comprising a movable arm part having DOFs *Φ* 111, *Ψ* 112, *Z* 113 and *Θ* 114, allowing instrument motions 107-110 as previously shown in Fig. 2, resulting in movements of the surgical instrument tip 122. The DOFs may be arranged such that there is a point on the surgical instrument 122 that does not move in space, termed the remote centre of motion (RCM) 125. By moving the base of the surgical arm, the movable arm part may be positioned such that the remote centre of motion 125 of the surgical instrument is positioned at the trocar. Respective actuators may be arranged to effect movement in all four DOFs 111-114.

As previously elucidated with reference to Fig. 1, the surgical robotic system may comprise a human machine interface for receiving operator commands, such as positioning commands, from a human operator, e.g., a surgeon or healthcare provider. In some embodiments, the human machine interface may comprise or be constituted by a motion controller such as a joystick. In some embodiments, the motion controller may be an external device with which the human machine interface or the processor subsystem 40 is configured to interface. In some embodiments, the motion controller may be comprised in the surgical robotic system as a further component, e.g., configured to interface with the processor subsystem 40 and/or the human machine interface 20. **Fig. 4** shows a joint diagram illustrating the kinematics of such a motion controller. Here, the motion controller is shown to have DOFs *Φₘ* 115, *Ψₘ* 116, *Zₘ* 117 and *Θₘ* 118. The human operator may provide operator commands, such as positioning commands, to directly control the movement of the movable arm part. In a specific example, the human operator may hold the motion controller at a gripper part, press the button 126 and move the gripper part of the motion controller in space.

It is noted that, in general, the operator commands received from the human operator may pertain to a set of DOFs which is different from the DOFs of the individual actuators. In other words, the control DOFs may be different from the actuator DOFs. The processor subsystem may be configured to convert, e.g., using a coordinate transformation, the operator commands into actuator control signals for the actuators. For example, the processor subsystem may receive and convert operator commands which pertain to the DOFs *Φₘ* 115, *Ψₘ* 116, *Zₘ* 117 and *Θₘ* 118 of Fig. 4 into actuator control signals pertaining to the DOFs *Φ* 111, *Ψ* 112, Z 113 and *Θ* 114 of Fig. 3.

**Fig. 5** shows a surgical instrument 119 in an eye 200. The tip of the surgical instrument 119 is also shown magnified. The tip of the surgical instrument 119 may be an operative tip in that the tip may be operatively involved in the intraocular procedure. For example, as shown in Fig. 5, the tip of the surgical instrument 119 may be or comprise a grasping device 122, being in the example of Fig. 5 and following a forceps comprising a pair of arms. As illustrated on the left hand side of Fig. 5, the surgical instrument 119 is shown inserted into the eye 200. The surgical instrument 119 may be approaching the retina 210 as illustrated in this figure, although this is merely exemplary and the invention is not limited thereto. In some embodiments, the surgical instrument 119 may approach another area, structure, or tissue layer of the eye, such as a capsular bag or a part of the eye's drainage system. The grasping device 122 of the surgical instrument 119 is illustrated on the right hand side of Fig. 5, magnified. In this example, a surgical target 123 in form of a retinal surface structure is located on the surface of the retina. In some embodiments, a sensor or sensor component 121, such as an optical fibre, may be integrated in or attached to the surgical instrument 119. The sensor 30 may comprise an optical fibre (as shown) coupled to an OCT sensor and configured to emit an optical beam 127 to capture cross-sectional images of the retina 210 and other structures within the eye. The optical fibre 121 may be recessed relative to the arms of the grasping device 122. Since the OCT probe may be longitudinally forward facing, the sensor data may be indicative of the longitudinal distance D to the retinal surface structure laterally forward of the grasping device 122, which may be considered to be 'beneath' or 'ahead' of the grasping device 122.

It will be appreciated that while the distance D may be larger than the distance of the distal tips of the grasping device 122 to the retinal surface structure, the distance D may be considered to underestimate the proximity of the grasping device to the retinal surface structure. However, since the dimensions of the grasping device 122 and its relative position to the optical fibre 121 or other sensor are typically known, the processor subsystem may determine the distance between the retinal surface structure to any part of the grasping device 122 using such known dimensions/relative positions.

It is noted that when the surgical instrument 119 is operated in the periphery of the eye, or in the vicinity of the posterior side of the lens in vitrectomy, the retina and/or the retinal surface structure may also be sideways proximate to the grasping device, and in some cases more proximate sideways than longitudinally. To account for such sideward proximity, the OCT probe may be a multi-directional probe with axial and side-viewing capability so as to be able to determine the distance to the retinal surface structure and/or retina also sideward to the grasping device 122.

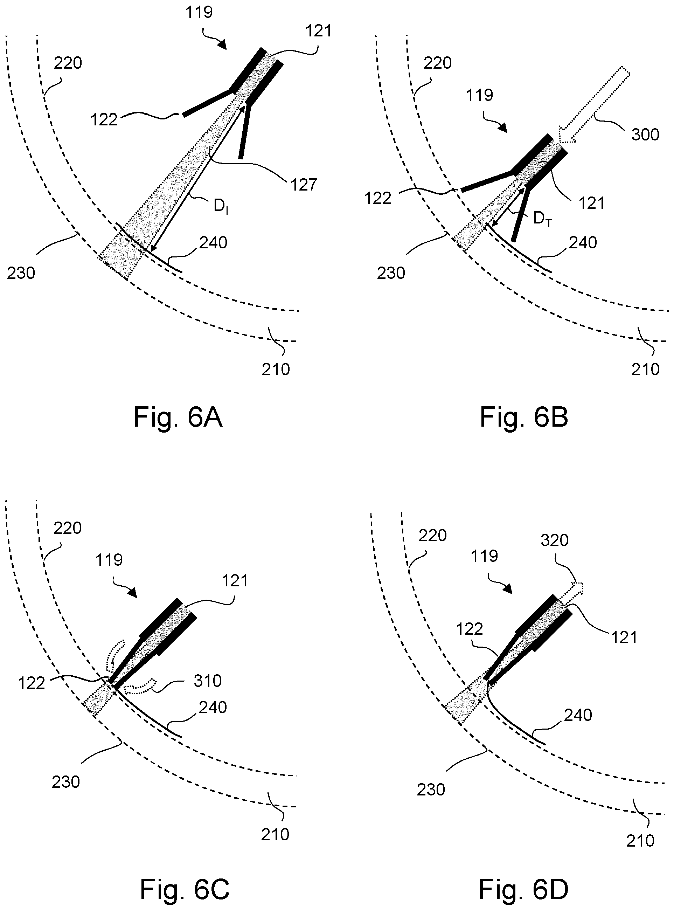

Figs. 6A-6F illustrate the use of the second operational mode within the context of a retinal peeling procedure. It will be appreciated, however, that this surgical procedure is merely exemplary and that the second operational mode may be used in various other surgical procedures and in general in various other situations.

**Fig. 6A** shows the surgical instrument 119 of Fig. 5 with its grasping device 122 positioned at an initial distance Dᵢ relative to a retinal surface structure 240 which is located on or within a retinal surface (with the retina 210 being shown in Fig. 6A and elsewhere as an upper layer 220 and lower layer 230). Such an initial position may for example be obtained by the human operator having directly controlled the position of the surgical instrument 119, for example while the processor subsystem of the surgical robotic system operates in the first operational mode, or as a result of the surgical robotic system having autonomously followed a certain trajectory. The initial distance Dᵢ may be too large to grasp the retinal surface structure 240, but may be large enough to reduce the risk of damaging the retinal surface to an acceptable degree. Such risk may otherwise be significant when the surgical instrument 119 is very proximate to the retina, e.g., due to the risk of inadvertent positioning errors of the surgical instrument 119 or external movement (e.g., due to periodic physiological movement). **Fig. 6B** shows the grasping device 122 having been repositioned to a target distance D_{T} to the retinal surface structure 240. The target distance D_{T} may a distance at which the retinal surface structure can be grasped. **Fig. 6C** shows the processor subsystem effecting a grasping action 310 with the grasping device 122 following the repositioning 300 of the grasping device previously shown in Fig. 6B. As may be known per se, the grasping action 310 may be carried out by actuating the grasping device 122, for example by actuating the arms of a forceps to mutually close, or by creating suction at an aspiration port of a micro vacuum pick, etc. It will be appreciated that the positional and grasping actions as illustrated in Figs. 6A-6C may be performed under the control of the human operator, for example while the processor subsystem of the surgical robotic system operates in the first operational mode. However, this is not a limitation, in that the actions may also be performed (semi)automatically by the processor subsystem.

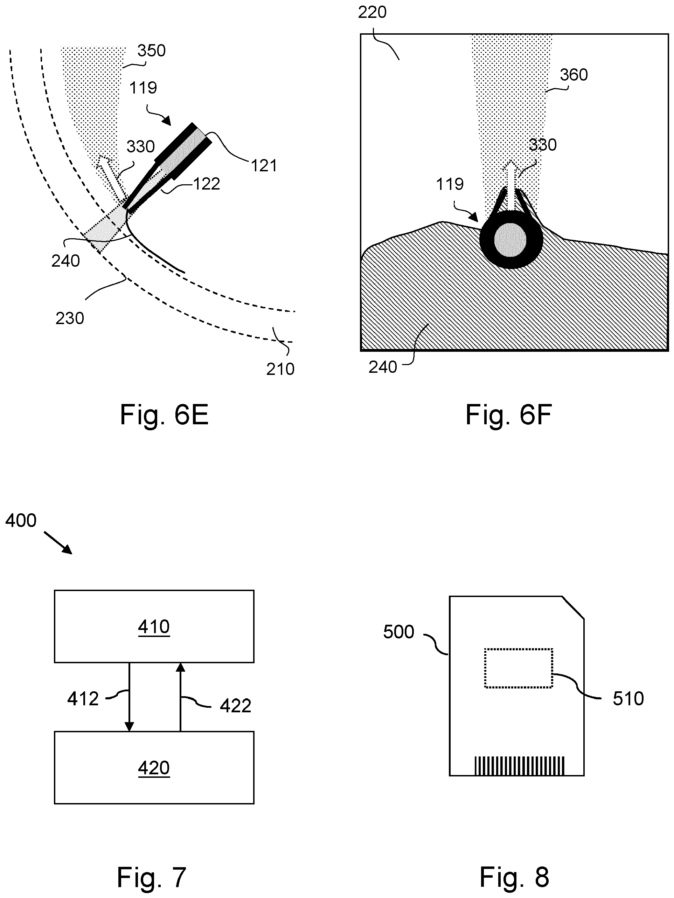

**Fig. 6D** shows a retreating movement 320 of the grasping device in which the surgical instrument 119 is moved longitudinally away from the retinal surface 220. Thereby, the retinal surface structure 240 may be pulled away from the surface of the retina 210, for example to prepare or start a peeling movement to remove the retinal surface structure. Before or after performing the retreating movement 320, the processor subsystem may switch to the second operational mode, for example in response to an operator command, or by having detected the grasping action and/or the retreating movement, etc. As shown in **Figs. 6E-6F**, as a result of the processor subsystem operating in the second operational mode, the human operator may continue to perform a peeling movement 330 but may be constrained in his/her control over the pose of the surgical instrument across one or a subset of the degrees of freedom. In particular, **Fig. 6E** shows the human operator being limited in his/her control over the pose of the surgical instrument by the longitudinal position of the surgical instrument, and in particular the longitudinal position of its operational tip, being limited to a longitudinal range 350. The longitudinal range 350 may for example be determined by the processor subsystem to avoid collision with the retina and to optimally support the peeling movement 330. **Fig. 6F**, which shows a top-down view of the surgical instrument 119, illustrates the human operator being limited in his/her control over the pose of the surgical instrument by the lateral position of the surgical instrument, and in particular the lateral position of its operational tip, being limited to a lateral range 360. The lateral range 360 may for example be determined by the processor subsystem to optimally support the peeling movement 330.

The operations depicted in Figs. 6A-6F may take place in the context of a vitreoretinal surgery in which a retinal membrane is peeled. During such retinal membrane peeling, there is a risk of injuring the patient's retina and causing trauma to the retina. This may happen on the one hand due to a collision between the surgical instrument and the retina, and on the other hand due to excessive force or traction being exerted on the retina which may be caused by pulling the membrane flap with the surgical instrument. It may be difficult for a surgeon to estimate the distance between the surgical instrument and the retina due to limited depth perception. During peeling, the membrane flap that is pulled may become longer and longer as more of the membrane is torn off. This may cause the instrument tip to move out of the microscope's field of view and the surgeon may no longer have the ability to monitor how close the surgical instrument is approaching the retina without readjustment of the microscope. The second operational mode may be used to assist the surgeon during such peeling. For example, the processor subsystem may exert partial or full control over the pose of the surgical instrument so as to guide the surgical instrument close above the retina in order to exert little force on the retina and while avoiding touching the retina. This may be explained as follows. Stress in the tissue due to the force exerted by the surgical instrument on the flap may be divided into normal and shear stress components. The membrane may tear most easily when the shear component is high. The shallower the angle between the force vector applied by the surgical instrument and the retina, the higher the shear component may be. Therefore, tearing may be most efficient when the surgical instrument is moved close to the retina. The processor subsystem may exert partial control of the instrument's pose to enforce or support such close movement of the surgical instrument to the retina.

In a specific example, the processor subsystem may exert full control over the longitudinal position of the surgical instrument during peeling. This way, the processor subsystem may control the longitudinal position to remain close to the retina to reduce traction on the retina while maintaining a minimum safety distance, for example 50µm. In another specific example, instead of fully controlling the longitudinal position of the surgical instrument, the processor subsystem may define a corridor in which the human operator is allowed to move the surgical instrument. This corridor may correspond to the longitudinal range 350 of Fig. 6E. The lower boundary of this corridor may be defined by a minimum safety distance, while the upper boundary of the corridor may be determined by a maximum distance at which the traction that the surgical instrument exerts on the retina is kept below a threshold. In such an example, the longitudinal position of the surgical instrument may be controlled by the human operator but limited or guided by the processor subsystem to remain within the corridor. In some examples, while moving the surgical instrument, the human operator may be provided with feedback if the surgical instrument approaches the boundaries of the corridor. The feedback may for example be haptic, visual and/or acoustic. For example, as visual feedback, it may be shown on a screen in which direction surgical instrument tool should be moved to stay within the corridor. Another example is that, as haptic feedback, the human machine interface may `push back' via haptic feedback in case the human operator operates the human machine interface to leave the corridor.

With continued reference to Fig. 6F, in some examples, the processor subsystem may be configured to determine a lateral trajectory for the surgical instrument, for example to automate and/or assist the lateral positioning of the surgical instrument, or a lateral corridor in which the human operator is allowed to move the surgical instrument. For example, the processor subsystem may determine the lateral trajectory or corridor by identifying wrinkles on the retina and determining the lateral trajectory or corridor based on the position and/or orientation of the wrinkles, for example to move the surgical instrument at a specific angle to the wrinkles, for example 90 degrees, which motion may then be followed by an arc-shaped motion. The position and/or orientation of the wrinkles may for example be determined based on preoperative and/or intraoperative sensor data. Another example is that the lateral trajectory or corridor may be determined using a biomechanical model modelling the tearing of the retinal membrane. The biomechanical model may be configured to receive as input the position of the surgical instrument relative to the retina, the detected layers of the retina, the observed wrinkle positions and orientations, etc. The processor subsystem may use the biomechanical model to, through simulation of the peeling and/or tearing, determine an optimal trajectory of the surgical instrument for low-traction yet efficient peeling. The trajectory may thus be optimized such that the traction exerted on the retina is relatively low, but at the same time, a large area of the membrane is peeled off and the peeling is done efficiently. This determined optimal trajectory may directly used to control the lateral position of the surgical instrument, used to define the lateral corridor for the surgical instrument, and/or may be indicated to the human operator.

With continued reference to the limitation to a positional range, it is noted that such a range limitation may take various forms. For example, the positional range limitation may be applied by limiting movement of the surgical instrument to a range of allowed directions, or by disallowing movement in a range of directions. As such, the positional range may be defined as a range of possible movement directions relative to a current position. The range of allowed movement directions may be updated by the processor subsystem based on a current position of the surgical instrument, for example to account for a curvature of the retina. In other examples, the positional range may be defined in absolute terms, e.g., in a coordinate system which is expressed in relation to the patient's retina. For example, the positional range may be defined based on geometric model of the retina, with the geometric model having been fitted by the processor subsystem to the retina of the patient at hand based on sensor data accessed by the processor subsystem. In general, the positional range may be one-dimensional range, e.g., defining a limitation across one positional DOF, but also a two-dimensional range, e.g., defining a limitation across two positional DOFs or a three-dimensional range, e.g., defining a limitation across three positional DOFs. The range may thus in some examples combine lateral and longitudinal limitations.

It is noted that while Figs. 6E-6F show a limitation to a positional range, the limiting of the adjustment of the pose by the human operator may additionally or alternatively involve applying limitations to the control of the orientation of the surgical instrument. For example, the human operator may be limited to a range of allowed orientations of the surgical instrument, or prevented from changing the orientation to a range of disallowed orientations. In another example, the control of the orientation of the surgical instrument may be taken over by the processor subsystem, allowing the human operator to focus on the positional control of the surgical instrument.

It is further noted that in some cases, the DOFs available to the human operator for control of the surgical instrument in the first operational mode may include DOFs which may relate to other operational aspects of the surgical instruments besides its position and orientation, such as DOFs relating to the execution of grasping actions, suction actions, or cutting actions. It will be appreciated that in the second operational mode, limitations may also be applied to such non-positional/orientational DOFs.

It is noted that while Figs. 6E-6F shown the positional range in form of a discrete range, the processor subsystem may alternatively or additionally limit the adjustment of the pose by the human operator to remain within a range by dampening the adjustment across the one or the subset of the degrees of freedom, for example by dampening excursions away from a reference point within the range or by dampening excursions beyond the range. Such dampening may also apply to orientational ranges.

In general, the limitation which is applied to the control by the human operator may be dependent on a relative position and/or relative orientation between the surgical instrument and an intraocular structure, such as the retina, and in other words, dependent on the pose of the surgical instrument relative to the intraocular structure. The processor subsystem may determine the relative position and/or relative orientation based on sensor data, for example based on sensor data obtained from one or more pre-operative and/or intraoperative sensors. For example, the intraoperative sensor data may be obtained from one or more intraocular and/or extraocular sensors as described elsewhere in this specification. By being able to determine the relative position and/or relative orientation of the surgical instrument, the processor subsystem may in the second operational mode exert control over one or more DOFs of the surgical robotic system so as to be able control a distance and/or orientation of the surgical instrument relative to the intraocular structure, for example across one or more positional and/or orientational DOFs. For example, the processor subsystem may control the distance and/or orientation to stay clear of the intraocular structure and/or to support a surgical action performed in respect of the intraocular structure.

In some examples, the processor subsystem may make use of a computational model of an intraocular structure which is pertinent in the intraocular procedure to exert the control over the one or more DOFs. For example, the computational model may be a geometric and/or a biomechanical model of the intraocular structure. Such a computational model may for example be used by the processor subsystem to control the distance and/or orientation of the surgical instrument to optimally support a surgical action. For example, the processor subsystem may be configured to use the computational model during the intraocular procedure to optimally support a peeling or similar action by limiting a magnitude of a force, or a directional component of the force, or a direction of the force, which is exerted by the surgical instrument onto the retina and/or the retinal membrane.

The geometric model may be parameterized, for example in terms of size and shape (e.g., curvature). The processor subsystem may be configured to determine one or more parameters of the geometric model based on sensor data acquired preoperatively or during the intraocular procedure. For example, the intraoperative sensor data may be obtained from one or more intraocular and/or extraocular sensors as described elsewhere in this specification. This way, a calibrated or patient-adapted model of the intraocular structure may be obtained, which may allow the processor subsystem to determine the positional and/or orientation of the surgical instrument in relation to that of the intraocular structure. The computational model may therefore represent a digital twin model of the patient's eye. In some embodiments, the sensor data obtained intraoperatively from a sensor used for the distance measurements may be used to determine the parameter(s) for the geometric model. For example, distance measurements at different positions within the eye may allow reconstruction of the curvature of the retinal surface structure. Additionally, or alternatively, extraocular sensor data from an extraocular sensor may be used, such as an optical coherence tomography, OCT, sensor, or a surgical microscope. Additionally, or alternatively, preoperative sensor data may be used, e.g., obtained from a planning phase.

The processor subsystem may switch from the first operational mode to the second operational mode, or in general from any other operational mode to the second operational mode, in response to various types of triggers. For example, the switch to the second operational mode may be triggered by a surgical action being performed with the surgical instrument, such as a grasping action, a suction action, or a cutting action, or a gesture being performed with the surgical instrument. The processor subsystem may also determine when to switch to the second operational mode based on internal switching logic which evaluates the computational model to determine when to switch to the second operational mode. In yet another example, the switch to the second operational model may be triggered by the human operator, for example in response to an operator command which comprises a request to switch or which is otherwise indicative of a need to switch. In some examples, the human operator may always be able to switch manually back to the first operational mode, e.g., to regain control over the pose of the surgical instrument from the processor subsystem.

In some examples, the processor subsystem may be configured to operate also further operational modes, e.g., in a third, fourth, etc, operational mode. Different operational model may allow for different levels of control to be exerted by the processor subsystem. Such exerting of control may be understood as limitations applying to the control by the human operator, but likewise as the human operator being supported in his/her control of the surgical instrument. The processor subsystem may thus assist the human operator by exerting partial control over the surgical instrument. Different operational modes may provide different levels of assistance.

An example of the use of the surgical robotic system outside of intraocular surgery may be one in which the surgical robotic system is configured to perform transoral laser microsurgery in the vocal cord region of the throat. For example, the surgical robotic system may be configured to treat or remove tumorous tissue using a laser delivery tool as the surgical instrument. In such examples, the first operational mode may be used to bring the laser delivery tool into the vicinity of the relevant tissue. The processor subsystem may then switch to the second operational mode to assist in human operator in controlling the distance between the tissue and the laser delivery tool and/or in controlling rotational degrees of freedom which determine the angle at which the laser beam is delivered onto the tissue. This way, the human operator may focus on the lateral position of the laser delivery tool or the laser spots while the surgical robotic system may assist in the control of the laser delivery tool by assuming poses at which the laser radiation is delivered at a preferably nearly constant distance to the tissue and preferably nearly perpendicular to the tissue. In such examples, an OCT-based distance sensor may be integrated in the laser delivery tool to provide tool-tissue distance feedback even in areas where the surgeon does not have a direct view onto the situs with a surgical microscope. In areas which are visible directly with a surgical microscope, a microscope-integrated tool-tissue distance sensing modality such as a stereo camera system or a microscope integrated OCT system may be used.

In another example, the surgical robotic system may be configured to deliver a cochlear implant to the cochlea using a surgical instrument. In such an example, one or more rotational degrees of freedom of the surgical instrument may be controlled in a second operational mode while inserting the implant into the cochlea. This way, the damage caused by collisions between the implant and the inner walls of the cochlea may be minimized. In such an example, positional feedback may be provided by an OCT probe embedded into the implant or the sheath holding the implant. In another example, force information may be acquired from a fiber Bragg grating (FBG) force sensor integrated into the cochlear implant.

In general, the processor subsystem described in this specification may comprise one or more (micro)processors which execute appropriate software. The software implementing the functionality of the processor subsystem may have been downloaded and/or stored in a corresponding memory or memories, e.g., in volatile memory such as RAM or in non-volatile memory such as Flash. Alternatively, the processor subsystem may be implemented in the form of programmable logic, e.g., as a Field-Programmable Gate Array (FPGA). Any input and/or output interfaces may be implemented by respective hardware and/or software interfaces. In general, the processor subsystem, input interfaces, and/or output interfaces may each be implemented in the form of a circuit or circuitry. The processor subsystem may also be implemented in a distributed manner, e.g., involving different devices or apparatus.

**Fig. 7** shows a method 400 of controlling a surgical robotic system during a surgical procedure. The method 400 may comprise, in a step 410, operating the processor subsystem of the surgical robotic system in a first operational mode as described elsewhere in this specification, and in response to a trigger 412, in a step 420, operating the processor subsystem of the surgical robotic system in a second operational mode as described elsewhere in this specification. In some examples, the processor subsystem may be triggered 422 to revert back to the first operational mode.

It is noted that any of the methods described in this specification, for example in any of the claims, may be implemented on a computer as a computer implemented method, as dedicated hardware, or as a combination of both. Instructions for the computer, e.g., executable code, may be stored on a computer-readable medium 500 as for example shown in **Fig. 8**, e.g., in the form of a series 510 of machine-readable physical marks and/or as a series of elements having different electrical, e.g., magnetic, or optical properties or values. The executable code may be stored in a transitory or non-transitory manner. Examples of computer-readable mediums include memory devices, optical storage devices, integrated circuits, servers, online software, etc. Fig. 8 shows by way of example a memory card 500.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A surgical robotic system (100) for use in an intraocular procedure, comprising:
- a surgical arm (80) comprising a movable arm part (82), wherein the movable arm part (82) comprises an end effector for holding a surgical instrument (119);
- an actuator subsystem (60) configured to actuate the movable arm part (82);
- a human machine interface (20) for receiving operator commands (22) from a human operator;
- a processor subsystem (40) configured to control the actuator subsystem (60) to control a pose of the surgical instrument (119) based on the operator commands (22);
wherein the surgical robotic system (100) is configured to operate in a first operational mode in which the human operator is enabled to adjust the pose of the surgical instrument (119) across multiple degrees of freedom by providing corresponding operator commands via the human machine interface (20) and wherein the processor subsystem (40) controls the actuator subsystem (60) to effect said adjustment of the pose of the surgical instrument (119) across the multiple degrees of freedom;
wherein the processor subsystem (40) is further configured to switch from the first operational mode to a second operational mode in response to a trigger, and in the second operational mode, exert control over the pose of the surgical instrument (119) across one or a subset of the degrees of freedom.

2. The surgical robotic system (100) according to claim 1, wherein the processor subsystem (40) is configured to exert the control by limiting the adjustment of the pose by the human operator across the one or the subset of the degrees of freedom.

3. The surgical robotic system (100) according to claim 2, wherein the processor subsystem (40) is configured to limit the adjustment of the pose by the human operator across the one or the subset of the degrees of freedom by disallowing said adjustment by the human operator or by limiting the adjustment to remain within a range (350, 360).

4. The surgical robotic system (100) according to claim 3, wherein the processor subsystem (40) is configured to limit the adjustment of the pose by the human operator to remain within the range (350, 360) by dampening the adjustment across the one or the subset of the degrees of freedom, for example by dampening excursions away from a reference point within the range (350, 360) or by dampening excursions beyond the range (350, 360).

5. The surgical robotic system (100) according to claim 3 or 4, wherein the processor subsystem (40) is configured to generate and output a sensory perceptible feedback signal which is indicative of the pose of the surgical instrument (119) in relation to the range, such as an audible signal, a haptic signal, and/or a visual signal.

6. The surgical robotic system (100) according to claim 2, wherein the processor subsystem (40) is configured to determine an extent to which to limit the adjustment of the pose by the human operator based on a relative position and/or relative orientation between the surgical instrument (119) and an intraocular structure (210, 240), such as the retina.

7. The surgical robotic system (100) according any one of claims 1 to 6, wherein the one or the subset of the degrees of freedom comprise one or more positional degrees of freedom, and wherein the processor subsystem (40) is configured to exert control over a position of the surgical instrument (119) across the one or more positional degrees of freedom, for example to control the surgical instrument (119) to remain within a positional range (350, 360), or to follow a trajectory, or to remain within a positional range of the trajectory.

8. The surgical robotic system (100) according to any one of claims 1 to 7, wherein the one or the subset of the degrees of freedom comprise one or more orientational degrees of freedom, and wherein the processor subsystem (40) is configured to exert control over an orientation of the surgical instrument (119) across the one or more orientational degrees of freedom, for example to control the orientation of the surgical instrument (119) relative to an intraocular structure (210, 240).

9. The surgical robotic system (100) according to any one of claims 1 to 8, wherein the processor subsystem (40) is configured to access pre-operative and/or intraoperative sensor data (32), and based on the sensor data (32), control a distance and/or orientation of the surgical instrument (119) relative to an intraocular structure (210, 240), for example across one or more positional degrees of freedom and/or one or more orientational degrees of freedom.

10. The surgical robotic system (100) according to any one of claims 1 to 9, wherein the processor subsystem (40) is configured to:
- access a computational model of an intraocular structure (210, 240) which is pertinent in the intraocular procedure, wherein the computational model is a geometric and/or a biomechanical model of the intraocular structure (210, 240); and
- exert the control across the one or the subset of the degrees of freedom based on the computational model.

11. The surgical robotic system (100) according to claim 10, wherein the processor subsystem (40) is configured to access pre-operative and/or intraoperative sensor data (32) and to calibrate the computational model using the sensor data (32).

12. The surgical robotic system (100) according to claim 10 or 11, wherein the processor subsystem (40) is configured to use the computational model during the intraocular procedure to limit a magnitude of a force, or a directional component of the force, or a direction of the force, which is exerted by the surgical instrument (119) onto or via the intraocular structure (210, 240).

13. The surgical robotic system (100) according to any one of claims to 1 to 12, wherein the processor subsystem (40) is configured to switch from the first operational mode to the second operational mode in response to at least one of:
- a surgical action being performed with the surgical instrument (119), such as a grasping action, a suction action, or a cutting action;
- a gesture being performed with the surgical instrument (119);
- switching logic indicating when to switch to the second operational mode, wherein the switching logic is configured to use a computational model of an intraocular structure which is pertinent in the intraocular procedure and evaluate the computational model to determine when to switch to the second operational mode.

14. The surgical robotic system (100) according to any one of claims 1 to 13, wherein the processor subsystem (40) is configured to switch from the first operational mode to the second operational mode in response to an operator command (22) which comprises a request to switch or which is otherwise indicative of a need to switch.

15. A computer-implemented method (400) for controlling a surgical robotic system during an intraocular procedure, wherein the surgical robotic system comprises:
a surgical arm comprising a movable arm part, wherein the movable arm part comprises an end effector for holding a surgical instrument;
- an actuator subsystem configured to actuate the movable arm part;
- a human machine interface for receiving operator commands from a human operator;
- a processor subsystem configured to control the actuator subsystem to control a pose of the surgical instrument based on the operator commands;
wherein the method comprises:
- in a first operational mode (410) of the surgical robotic system:
receiving operator commands from the human machine interface to adjust the pose of the surgical instrument across multiple degrees of freedom, and
controlling the actuator subsystem to effect said adjustment of the pose of the surgical instrument across the multiple degrees of freedom;
- switching (412) from the first operational mode to a second operational mode in response to a trigger;
- in the second operational mode (420), exerting control over the pose of the surgical instrument across one or a subset of the degrees of freedom.

16. A transitory or non-transitory computer-readable medium (500) comprising data (510) representing a computer program, the computer program comprising instructions for causing a processor system to perform the method according to claim 15.
